# EUROPEAN PATENT APPLICATION

(11) **EP 3 954 282 A1**
(43) Date of publication of application: **16.02.2022**
(21) Application number: 20788638.3
(22) Date of filing: 10.04.2020
(51) Int. Cl.: A61B 5/04, A61N 1/36, A61N 1/375

(54) **WIRE ARRAY AND BIO-IMPLANTABLE DEVICE COMPRISING SAME**

(30) Priority: 10.04.2019 KR 20190041951; 03.01.2020 KR 20200000692
(71) Applicant: Todoc Co., Ltd., Seoul 08394 (KR)
(72) Inventor: MIN, Kyou Sik, Suwon-si Gyeonggi-do 16493 (KR)
(74) Representative: Fuchs Patentanwälte Partnerschaft mbB
(86) International application number: PCT/KR2020/004877
(87) International publication number: WO 2020/209659

(57) **Abstract**

Proposed is an electric wire array including: a substrate portion; and a plurality of electric line units being arranged on top of the substrate portion and being stacked on top of each other in a thickness direction, wherein the plurality of electric wire units each comprise: a plurality of electric wires being arranged on top of the substrate portion in such a manner to extend in a length direction; and a plurality of electrode holes being positioned in respective first end portions, respectively, of the plurality of electric wires, and being exposed, wherein the plurality of electrode holes are arranged in such a manner as to be positioned a distance apart from each other along the length direction and being sequentially arranged in a successive manner along the thickness direction, and wherein the thickness direction is a direction from the substrate portion to the electric wire unit, and the length direction is a direction vertical to the thickness direction.

## Description

### [Technical Field]

Embodiments relate to an electric wire and a device implantable into a living body, the device including the electric wire array.

### [Background Art]

Structures that are worn on or implanted into a living body to convert a biochemical reaction and a biological signal into an electric signal and then collect the result or to apply an electric signal for living body stimulus to a nervous tissue, are collectively referred to as an implantable device. A device implantable into a living body, which is equipped with a micro-electric wire array, is also referred to as a micro-electric wire array package.

A device implantable into a living body, such as a stimulator implantable into a living body, a detector implantable into a living body, a cardiac pacemaker, a neural prosthesis, or a neuro-modulation device, operates with at least one portion thereof being implanted into the living body, that is, being inserted into the living body. For this reason, a sealed state of the implantable device is very important. In a case where the sealed state of the implantable device is poor, a body fluid may leak into an electronic circuit present within the implantable device. This leakage may cause various problems, such as a failure or malfunction in the implantable device and the shortened lifetime thereof.

In addition, the micro-electric wire array package may have a plurality of electrodes that function in various ways in such a manner as to transmit and receive various biological signals. In this case, the plurality of electrodes may be connected by a plurality of electric wires to an internal electronic circuit.

For this reason, there occurs a problem in that a resistance in the electric wire and a size thereof increase according to respective positions of the plurality of electrodes.

### [DISCLOSURE]

### [Technical Problem]

An objective of embodiments is to provide an electric wire array in which an electric wire is optimally positioned within a pad portion and an implantable device equipped with the electric wire array.

In addition, another objective thereof is to provide an electric wire array having an improved electric property and an implantable device equipped with the electric wire array.

In addition, still another objective thereof is to provide an electric wire array having an improved structural property and an implantable device equipped with the electric wire array.

Other problems to be solved and other objectives and advantageous effects would be derived from technical solutions to the problem and embodiments, which will be described below.

### [Technical Solution]

According to an embodiment, there is provided an electric wire array including: a substrate portion; and a plurality of electric line units being arranged on top of the substrate portion and being stacked on top of each other in a thickness direction, wherein the plurality of electric wire units each include: a plurality of electric wires being arranged on top of the substrate portion in such a manner to extend in a length direction; and a plurality of electrode holes being positioned in respective first end portions, respectively, of the plurality of electric wires, and being exposed, wherein the plurality of electrode holes are arranged in such a manner as to be positioned a distance apart from each other along the length direction and being sequentially arranged in a successive manner along the thickness direction, and wherein the thickness direction is a direction from the substrate portion to the electric wire unit, and the length direction is a direction vertical to the thickness direction.

In the electric wire array, an entire thickness of a first end portion of the plurality of electric wires may be smaller than an entire thickness of a second end portion of the plurality of electric wires.

In the electric wire array, a maximum width of the plurality of electric wires may be positioned more adjacent to the second end portion than to the first end portion.

In the electric wire array, the plurality of electric wire units each may include: a first electric wire unit; and a second electric wire unit arranged on top of the first electric wire unit, the first electric wire unit may include a plurality of first electric wires having a plurality of first electrode holes, respectively, and the second electric wire unit may include a plurality of second electric wires having a plurality of second electrode holes, respectively.

In the electric wire array, the plurality of first electrode holes may overlap in the length direction, and the plurality of second electrode holes may overlap in the length direction.

In the electric wire array, the plurality of first electric wires may be arranged in such a manner as to be positioned a distance apart from each other, and the plurality of second electric wires may be arranged in such a manner as to be positioned a distance apart from each other.

In the electric wire array, an entire thickness of a first end portion of the plurality of electric wires may be smaller than an entire thickness of a second end portion of the plurality of electric wires.

In the electric wire array, the plurality of electric wires may include: a first thickness section having a maximum thickness; and a second thickness section other than the first thickness section, and a length of the first thickness section may be greater than a length of the second thickness section.

In the electric wire array, the electrode hole may collect a biological signal.

According to an embodiment, there is provided an implantable device including: a body portion; and an electric wire array electrically connected to the body portion, wherein the electric wire array includes; a substrate portion; and a plurality of electric line units being arranged on top of the substrate portion and being stacked on top of each other in a thickness direction, wherein the plurality of electric wire units each include: a plurality of electric wires being arranged on top of the substrate portion in such a manner to extend in a length direction; and a plurality of electrode holes being positioned in respective first end portions, respectively, of the plurality of electric wires, and being exposed, wherein the plurality of electrode holes are arranged in such a manner as to be positioned a distance apart from each other along the length direction and being sequentially arranged in a successive manner along the thickness direction, wherein the body portion includes: pad portions electrically connected to the plurality of electric wire units, respectively; and a circuit unit including terminals electrically connected to the pad portions, respectively, and wherein the thickness direction is a direction from the substrate portion to the electric wire unit, and the length direction is a direction vertical to the thickness direction.

### [Advantageous Effects]

According to embodiments, an electric wire array in which an electric wire is optimally positioned and an implantable device equipped with the electric wire array can be realized.

In addition, an electric wire array having an improved electric property and an implantable device equipped with the electric wire array can be manufactured.

In addition, an electric wire array having an improved structural property and an implantable device equipped with the electric wire array.

Various useful advantages and effects of the present invention are not limited to those mentioned above and would be more easily understandable from detailed descriptions of the embodiments of the present invention.

### [Description of Drawings]

FIG. 1 is a perspective view illustrating an implantable device according to a first embodiment of the present invention;
FIG. 2 is an exploded perspective view illustrating the implantable device according to the first embodiment of the present invention;
FIG. 3 is a view illustrating the implantable device according to the first embodiment of the present invention, viewed from above;
FIG. 4 is a view illustrating the implantable device according to the first embodiment of the present invention, viewed from below;
FIG. 5 is a view illustrating an electric wire within an electric wire array according to the first embodiment of the present invention;
FIG. 6 is an enlarged view illustrating portion A in FIG. 5;
FIG. 7 is a plane view illustrating each electric wire within the electric wire array according to the first embodiment of the present invention;
FIG. 8 is an enlarged view illustrating portion B in FIG. 7;
FIG. 9 is an enlarged view illustrating portion C in FIG. 7;
FIG. 10 is a side view of the electric wire within the electric wire array according to the first embodiment of the present invention;
FIG. 11 is a view illustrating an electric wire within an electric wire unit according to a second embodiment of the present invention;
FIG. 12 is a plane view illustrating each electric wire within the electric wire array according to the second embodiment;
FIG. 13 is an enlarged view illustrating portion D in FIG. 12; and
FIG. 14 is a view illustrating an electric wire within an electric wire array according to a third embodiment of the present invention.

### [Mode for Invention]

Various modifications can be made to the present invention, and thus various embodiments thereof can be realized. Accordingly, specific embodiments will be described below with reference to the drawings. However, this description of the specific embodiments is not intended to limit the present invention thereto. All alterations, equivalents, and substitutes that are included within the scope of the technical idea of the present invention should be understood as falling within the scope of the present invention.

The terms first, second, and so forth may be used to describe various elements without imposing any limitation on the meanings thereof. These terms are used only to distinguish one element from another. For example, a first constituent element may be expressed as a second constituent element without departing from the scope of the present disclosure. In the same manner, the second constituent element may also be expressed as the first constituent element. The phrase "and/or" is used to join two words, phrases, or sentences or to refer to one of the two words, phrases, or sentences.

It should be understood that, when a constituent element is referred to as being "coupled to" or "connected to" a different constituent element, this means that the constituent element may be directly coupled to or directly connected to the different constituent element or means that an intervening constituent element may be present therebetween. In contrast, it should be understood that, when a constituent element is referred to as being "directly coupled to" or "directly connected to" a different constituent element, this means that no intervening constituent element is present therebetween.

The terms used in the present application are only to describe a specific embodiment without being intended to impose any limitation on the present invention. The indefinite article "a/an" is used to mean one or more, not only one, except as distinctively expressed in context. The term "include," "have" or the like in the present application is intended to indicate that a feature, a number, a step, an operation, a constituent element, a component, or combinations of these, which is described in the specification, is present, and thus should be understood not to pre-exclude the possibility that one or more other features, numbers, steps, operations, constituent elements, components, or combinations of these will be present or added.

Unless otherwise defined, all terms including technical or scientific terms, which are used in the present specification, have the same meanings as are normally understood by a person of ordinary skill in the art to which the present invention pertains. The term as defined in a dictionary that is in general use should be construed as having the same meaning as interpreted in context in the relevant technology, and, unless otherwise explicitly defined in the present specification, is not construed as having an ideal meaning or an excessively-formal meaning.

Embodiments will be described in detail below with reference to the accompanying drawings. Like constituent elements or corresponding constituent elements are given like reference numerals, and only one of the like constituent elements or the corresponding constituent elements is described.

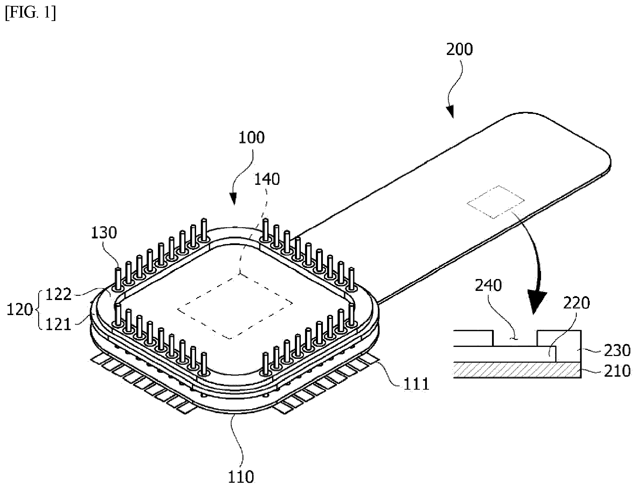

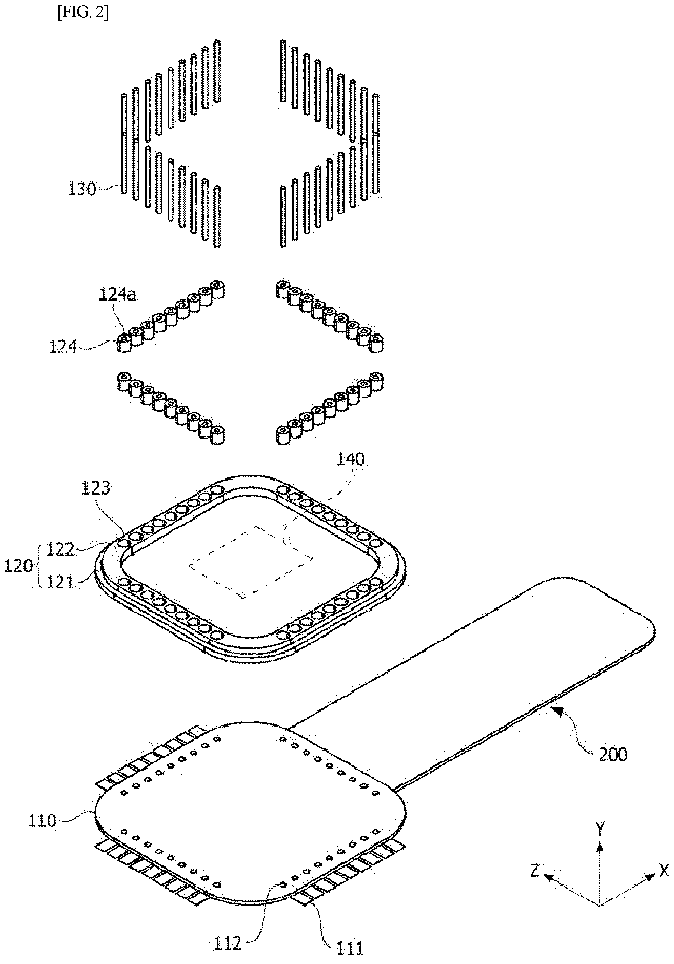

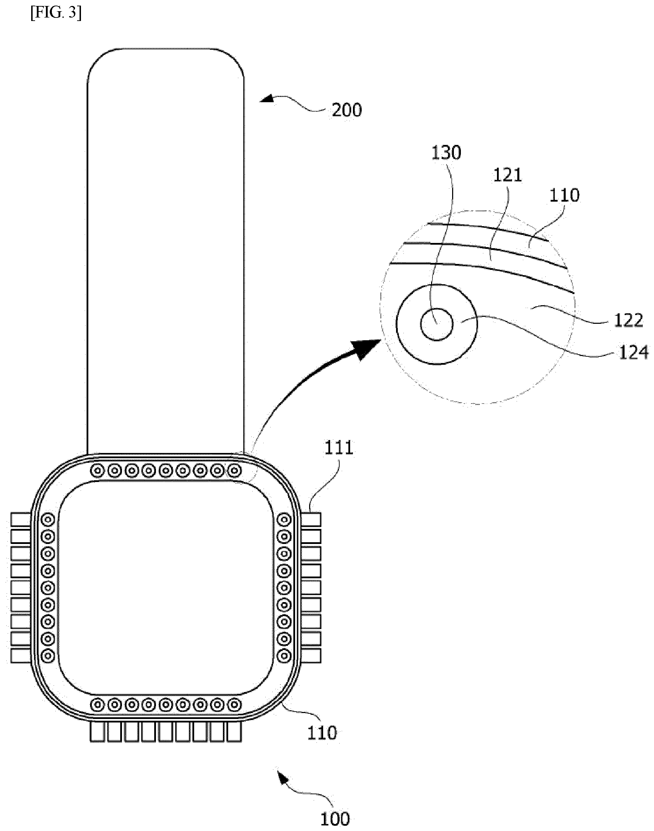

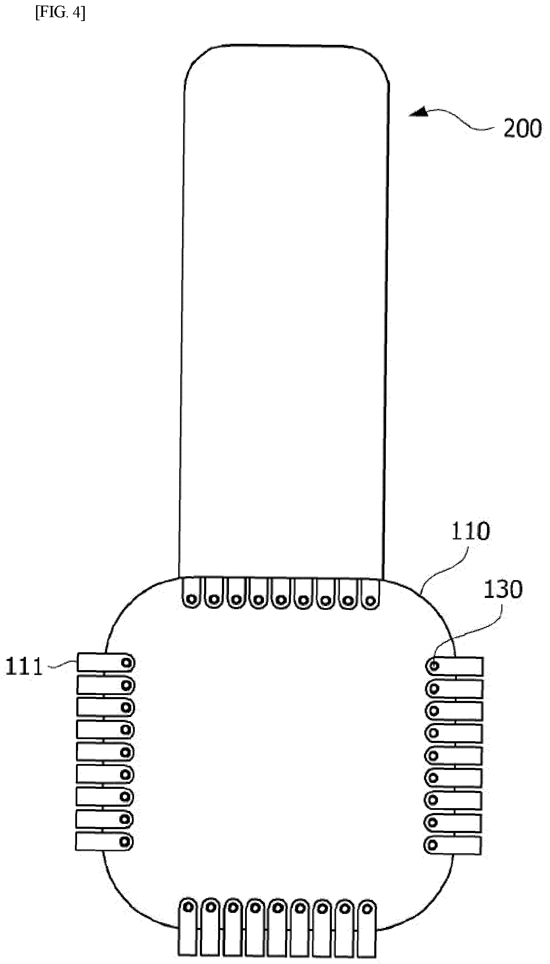

FIG. 1 is a perspective view illustrating an implantable device according to a first embodiment of the present invention. FIG. 2 is an exploded perspective view illustrating the implantable device according to the first embodiment of the present invention. FIG. 3 is a view illustrating the implantable device according to the first embodiment of the present invention, viewed from above. FIG. 4 is a view illustrating the implantable device according to the first embodiment of the present invention, viewed from below.

An implantable device 10 according to the first embodiment may include a main body 100 and an electric wire array 200 with reference to FIGS. 1 to 4.

Specifically, the main body 100 may include a base portion 110, a combination portion 120, a penetration pin 130, and a circuit unit 140. The base portion 110 is arranged in such a manner as to constitute a lower portion of the main body 100. The combination portion 120 is arranged on top of the base portion 110. The penetration pin 130 connects the combination portion 120 and the base portion 110 to each other. The circuit unit 140 is arranged within the main body 100.

The base portion 110 may be positioned in such a manner as to constitute one side of the main body 100. In the first embodiment, the base portion 110 may be positioned in such a manner as to constitute a lowermost portion of the main body 100. A cross-section of the base portion 110 may have various shapes, such as a rectangle, a circle, an ellipse, and the like, but is not limited to these shapes.

In addition, the base portion 110 may include a pad portion 111 and a base hole 112. The pad portion 111 is arranged in such a manner as to constitute a lower portion of the base portion 110 and a first end portion thereof is exposed to outside of the base portion 110. The base hole 112 is arranged along an edge of the base portion 110.

The pad portion 111 may be arranged in such a manner as to constitute the lowermost portion of the base portion 110. The base hole 112 may be arranged along the edge of the base portion 110. A plurality of pad portions 111 and a plurality of base holes 112 may be provided. The number of the pad portions 111 may be the same as the number of the base holes 112. Accordingly, the pad portion 111 may be electrically connected to the circuit unit 140 and the electric wire array 200 within the main body 100. In the first embodiment, a first end portion of the pad portion 111 is electrically connected to the circuit unit 140, and thus an electric signal may be provided to the pad portion 111 in order to operate the implantable device 10. Then, a second end portion of the pad portion 111 is connected to the electric wire array 200, and thus a living body stimulus signal generated in the circuit unit 140 may be finally provided to a living body with which an electrode hole 240 in the electric wire array 200 is brought into contact. In addition, in the reverse direction, a living body signal may be provided to the circuit unit 140 through the electrode hole 240 in the electric wire array 200.

In addition, the pad portion 111 and the base hole 112 may be combined with each other. For example, the pad portion 111 may include a pad hole in an area thereof that is brought into contact with the base portion 110. The pad hole is a through-hole and may be arranged at a position corresponding to the base hole 112. Accordingly, the penetration pin 130 passes through the base hole 112 and the pad hole, and thus may combine the base portion 110 and the combination portion 120 with each other.

The combination portion 120 may be arranged on top of the base portion 110. An area of the combination portion 120 may be smaller than that of the base portion 110. Accordingly, the combination portion 120 may be easily supported on the base portion 110, and thus the reliability of the implantable device can be improved.

According to the first embodiment, the combination portion 120 may include a first combination portion 121 and a second combination portion 122. The first combination portion 121 may be arranged on top of the base portion 110. That is, the first combination portion 121 may be positioned between the second combination portion 122 and the base portion 110. In addition, an area of the first combination portion 121 is smaller than that of the base portion 110 and may be greater than that of the second combination portion 122. Accordingly, the structural stability may be further improved.

The first combination portion 121 and the second combination portion 122 each may include a combination hole 123. The combination hole 123 may be a through-hole.

In addition, a plurality of combination holes 123 may be provided, and the number of the combination holes 123 may be the same as the number of the above-described pad portion 111 or the number of the above-described base hole 112.

The first combination portion 121 and the second combination portion 122 may be arranged to extend in a successive manner along the edge of the base portion 110. Accordingly, the first combination portion 121 and the second combination portion 122 may include a cavity 125 inside. The cavity 125 is an empty space. Accordingly, the first combination portion 121 and the second combination portion 122 can easily protect the circuit unit 140, arranged in the cavity 125, from the outside.

An area of the combination hole 123 may be greater than that of the above-described base hole 1112. A fixation member 124 may be arranged within the combination hole 123.

The fixation member 124 may include a fixation hole 124a. An area of the fixation hole 124a may be the same as that of the base hole 112. As is the case with the combination hole 123, a plurality of fixation holes 124a may be provided. The plurality of fixation holes 124a may be positioned in such a manner as to correspond to the plurality of base holes 112, respectively. Accordingly, with the penetration pin 130, the combination portion 120 and the base portion 110 may be held in place. In addition, each terminal of the circuit unit 140 arranged in the cavity 125 within the combination portion 120 may be electrically connected to the pad portion 111 through the penetration pin 130. Therefore, the implantable device 10 according to the first embodiment can ensure the increased tightening force and the ease of electric connection.

A plurality of penetration pins 130 may be provided. As is the case with the base hole 112, the plurality of penetrations pins 130 may be arranged along respective edges of the base portion 110 and the combination portion 120. The respective numbers of the base holes 112, the combination holes 123, the fixation holes 124a, and penetration pins 130 may vary with the number of the terminals (not illustrated) of the circuit unit 140.

The circuit unit 140 performs various functions, such as signal processing and communication, that are necessary for normal operation of the device 10 implantable into a living body. Generally, the circuit unit 140 is configured with one or more functional modules.

In addition, the circuit unit 140, as described above, may include a terminal electrically connected to a first end portion of the pad portion 111. Thus, through the terminal, a signal (for example, electric current, voltage, or the like) may be provided to the circuit unit 140 or may be provided from the circuit unit 140 in the reverse direction (to the electric wire array 200).

The electric wire array 200 may be arranged outward from the base portion 110. In the first embodiment, the electric wire array 200 is brought into contact with the base portion 110 and may be connected to a first end portion of the pad portion 111.

Specifically, the electric wire array 200 may include a substrate portion 210, an electric wire unit 220, a cover portion 230, and the electrode hole 240. The substrate portion 210 is arranged in such a manner as to constitute a lower portion of the electric wire array 200. The electric wire unit 220 is arranged on top of the substrate portion 210. The cover portion 230 covers the substrate portion 210 and the electric wire unit 220. The electrode hole 240 is exposed at a first end portion of the electric wire unit 220 in such a manner as to be brought into contact with the living body.

The substrate portion 210 is arranged in such a manner as to constitute the lower portion of the electric wire array 200 and may support the electric wire unit 220 and the like that are positioned thereon. The substrate portion 210 may be formed of a material not harmful to the living body. For example, the substrate portion 210 may be formed of polymer, ceramic, or the like, but is not limited to these materials.

The electric wire unit 220 may be arranged on top of the substrate portion 210. The electric wire unit 220 may be configured with a plurality of electric wires. For example, the electric wire unit 220 may extend in a first direction (the X-axis direction). The first direction (the X-axis direction) here is defined as a direction in which the plurality of electric wires extend. That is, the first direction is a direction from a first end portion of the electric wire to a second end portion thereof. A second direction (the Y-axis direction) may be a thickness direction, as a direction vertical to the first direction (the X-axis direction). The second direction is a direction in which a plurality of electric wires, that is, respective electric wires for channels are stacked on top of each other (for example, a direction from a first electric wire unit to a second electric wire unit). Accordingly, the second direction (the Y-axis direction) may correspond to a direction from the base portion 110 to the combination portion 120. A third direction (the Z-axis direction) may be a thickness direction, as a direction vertical to both the first direction (the X-axis direction) and the second direction (the Y-axis direction).

A plurality of electric wire units 220 the number of which corresponds to the number of the terminals of the circuit units 140 may be provided. In addition, the electric wire unit 220 may be arranged in such a manner as to extend from the pad portion 111 up to the electrode hole 240 brought into contact with the living body. The electric wire unit 220 may be configured using various circuit patterning methods, such as semiconductor fixation, but is not limited to these methods.

The cover portion 230 may cover the substrate portion 210 and the electric wire unit 220. In the first embodiment, the cover portion 230 may cover the electric wire unit 220 and thus may protect the electric wire unit 220 from an outside material. The cover portion 230 may be brought into contact with the living body. For this reason, the cover portion 230 may be formed of a material not harmful to the living body. For example, the cover portion 230 may be formed of a material, such as polymer, but is not limited to this material.

The cover portion 230 may cover one portion of the electric wire unit 220 in such a manner that a first end portion of the electric wire unit 220 is exposed from the cover portion 230. In this case, the electrode hole 240 may be a portion that is exposed at the electric wire unit 220 by the cover portion 230 and thus is brought into contact with the living body. An additional electrode pad or the like that is brought into contact with the living body may be arranged in the electrode hole 240. That is, an electrode or the like of which a surface area is increased in such a manner as to facilitate signal transmission/reception between the living body and the electric wire unit 220 may be arranged in the electrode hole 240.

In addition, in a case where the implantable device 10 collects the living body signal, the electrode hole 240 may serve as an entrance for the collected living body signal. Furthermore, in a case where the implantable device 10 transmits a living body stimulus signal to the living body, the electrode hole 240 may serve as an exit for the living body stimulus signal. A plurality of electrode holes 240 may be provided. The number of the electrode holes 240 may be the same as the number of the pad portions 111 and the number of the terminals of the circuit unit 140.

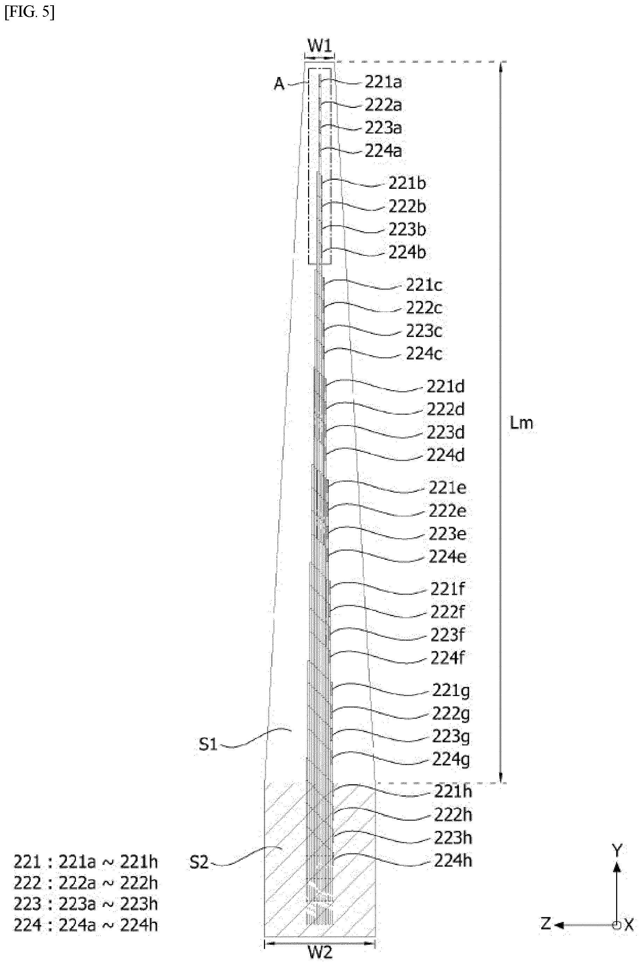

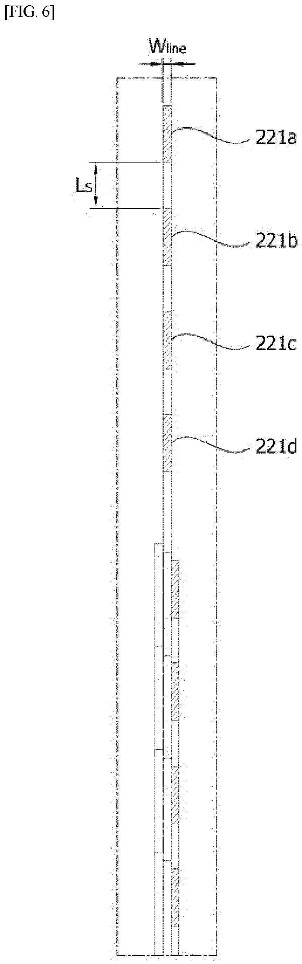

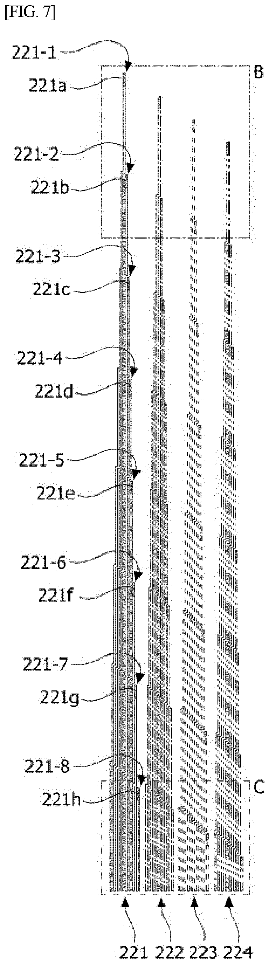

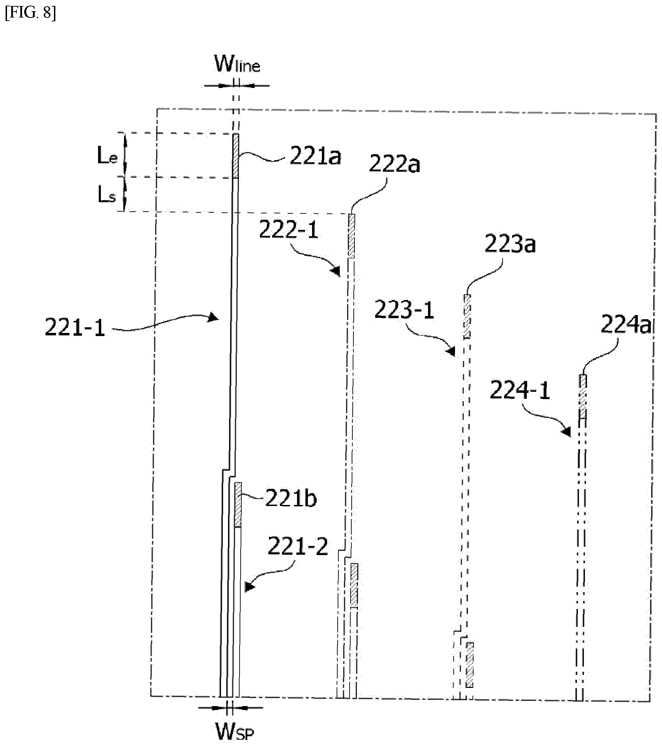

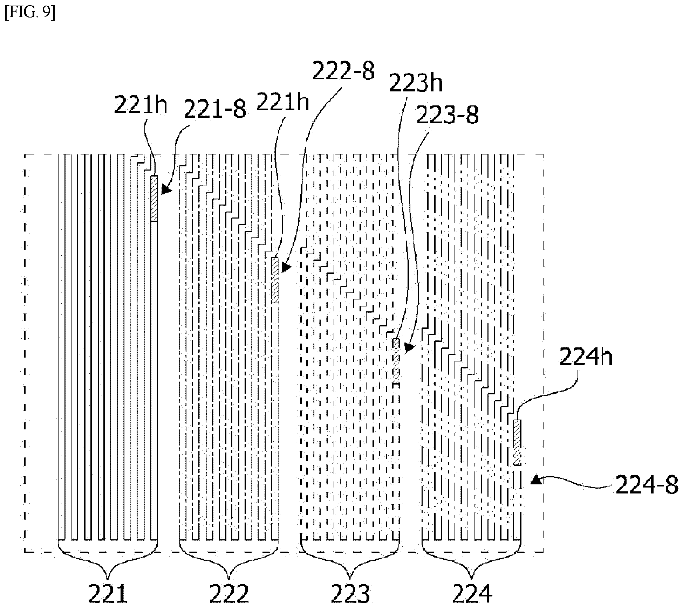

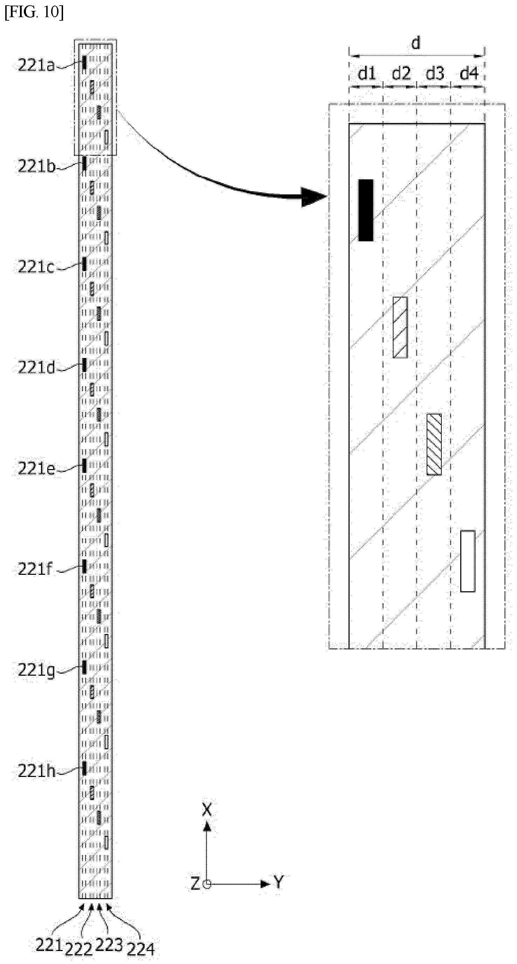

FIG. 5 is a view illustrating an electric wire within the electric wire array according to the first embodiment of the present invention. FIG. 6 is an enlarged view illustrating portion A in FIG. 5. FIG. 7 is a plane view illustrating each electric wire within the electric wire array according to the first embodiment of the present invention. FIG. 8 is an enlarged view illustrating portion B in FIG. 7. FIG. 9 is an enlarged view illustrating portion C in FIG. 7. FIG. 10 is a side view of the electric wire within the electric wire array according to the first embodiment of the present invention.

With reference to FIGS. 5 to 10, in the first embodiment, the implantable device may include a plurality of electric wire units 220, and each of the plurality of electric wire units, as described above, may include a plurality of wires. In addition, the plurality of electric wires may include a plurality of electrode holes, respectively. Each electrode hole is positioned in a first end portion of the electric wire. In this case, the plurality of electric wires may be arranged in such a manner as to extend in the first direction (the X-axis direction).

In the first embodiment, the electric wire unit 220 may include a first electric wire unit 221, a second electric wire unit 222, a third electric wire unit 223, and a fourth electric wire unit 224. The number of the electric wire units 220 may correspond to the number of channels of the circuit unit. For example, the circuit unit may have a plurality of terminals performing different functions, as one channel. A plurality of channels, each serving as the plurality of terminals, may be provided. In the first embodiment, in a case where the number of the channels of the circuit unit is 4, the electric wire unit 220, as described above, may include the first to fourth electric wire units 221 to 224. A description is provided below based on this case. Each electric wire unit may include a plurality of electric wires. However, in the present specification, a description is provided on the assumption of 8 electric wires per electric wire unit.

Specifically, the first to fourth electric wire units 221 to 224 each may have 8 electric wires. Accordingly, the first electric wire unit 221 may include (1-1)-st to (1-8)-st electric wires 221-1 to 221-8. Likewise, the second electric wire unit 222 may include (2-1)-nd to (2-8)-nd electric wires 222-1 to 222-8. The third electric wire 223 may include (3-1)-rd to (3-8)-rd electric wires 223-1 and 223-8. The fourth electric wire unit 224 may include (4-1)-th to (4-8)-th electric wires 224-1 and 224-8.

The above-described electrode hole may be arranged in a first end portion of each electric wire. Each electric wire may be arranged in such a manner as to extend from each electrode hole to the pad portion.

For example, a (1-1)-st electric wire 221-1 may include a (1-1)-st electrode hole 221a. The (1-2)-st electric wire 221-2 may include a (1-2)-st electrode hole 221b. The (1-3)-st electric wire 221-3 may include a (1-3)-st electrode hole 221c. The (1-4)-st electric wire 221-4 may include a (1-4)-st electrode hole 221d. The (1-5)-st electric wire 221-5 may include a (1-5)-st electrode hole 221e. The (1-6)-st electric wire 221-6 may include a (1-6)-st electrode hole 221f. The (1-7)-st electric wire 221-7 may include a (1-7)-st electrode hole 221g. The (1-8)-st electric wire 221-8 may include a (1-8)-st electrode hole 221h.

The (2-1)-nd electric wire 222-1 may include a (2-1)-nd electrode hole 222a. The (2-2)-nd electric wire 222-2 may include a (2-2)-nd electrode hole 222b. The (2-3)-nd electric wire 222-3 may include a (2-3)-nd electrode hole 222c. The (2-4)-nd electric wire 222-4 may include a (2-4)-nd electrode hole 222d. The (2-5)-nd electric wire 222-5 may include a (2-5)-nd electrode hole 222e. The (2-6)-nd electric wire 222-6 may include a (2-6) electrode hole 222f The (2-7)-nd electric wire 222-7 may include a (2-7)-nd electrode hole 222g. The (2-8)-nd electric charge 222-8 may include a (2-8)-nd electrode hole 222h.

The (3-1)-rd electric wire 223-1 may include a (3-1)-rd electrode hole 223a. The (3-2)-rd electric wire 223-2 may include a (3-2)-rd electrode hole 223b. The (3-3)-rd electric wire 223-3 may include a (3-3)-rd electrode hole 223c. The (3-4)-rd electric wire 223-4 may include a (3-4)-rd electrode hole 223d. The (3-5)-rd electric wire 223-5 may include a (3-5)-rd electrode hole 223e. The (3-6)-rd electric wire 223-6 may include a (3-6)-rd electrode hole 223f The (3-7)-rd electric wire 223-7 may include a (3-7)-rd electrode hole 223g. The (3-8)-rd electric wire 223-8 may include a (3-8)-rd electrode hole 223h.

The (4-1)-th electric wire 224-1 may include a (4-1)-th electrode hole 224a. The (4-2)-th electric wire 224-2 may include a (4-2)-th electrode hole 224b. The (4-3)-th electric wire 224-3 may include a (4-3)-th electrode hole 224c. The (4-4)-th electric wire 224-4 may include a (4-4)-th electrode hole 224d. The (4-5)-th electric wire 224-5 may include a (4-5)-th electrode hole 224e. The (4-6)-th electric wire 224-6 may include a (4-6)-th electrode hole 224f The (4-7)-th electric wire 224-7 may include a (4-7)-th electrode hole 224g. The (4-8)-th electric wire 224-8 may include a (4-8)-th electrode hole 224h.

The electrode hole arranged in each electric wire may be positioned in a first end portion thereof, which is an outermost portion in the first direction (the X-axis direction). That is, the (1-1)-st electrode hole 221a may be arranged in such a manner as to be positioned the farthest away from the pad portion. In the first electric wire unit 221, the (1-1)-st electric wire 221-1 may have the greatest length. The length here means a length in the first direction (the X-axis direction), and the first direction (the X-axis direction) may be a length direction. The (1-2)-st electrode hole 221b, the (1-3)-st electrode hole 221c, the (1-4)-st electrode hole 221d, the (1-5)-st electrode hole 221e, the (1-6)-st electrode hole 221f, the (1-7)-st electrode hole 221g, and the (1-8)-st electrode hole 221h are arranged in such a manner as to be positioned distances away from the pad portion, and a separation distance from the pad portion may be decreased in this order. That is, the (1-8)-st electrode hole 221h may be arranged in such a manner as to be positioned the closest to the pad portion. In the first electric wire unit 221, the (1-8)-st electric wire 221-8 may have the smallest length.

Likewise, the (2-1)-nd electrode hole 222a may be arranged in such a manner as to be positioned the farthest away from the pad portion. That is, in the second electric wire unit 222, the (2-1)-nd electric wire 222-1 may have the greatest length. The (2-2)-nd electrode hole 222b, the (2-3)-nd electrode hole 222c, the (2-4)-nd electrode hole 222d, the (2-5)-nd electrode hole 222e, the (2-6) electrode hole 222f, the (2-7)-nd electrode hole 222g, and the (2-8)-nd electrode hole 222h are arranged in such a manner as to be positioned distances away from the pad portion, and a separation distance from the pad portion may be decreased in this order. That is, the (2-8)-nd electrode hole 222h may be arranged in such a manner as to be positioned the closest to the pad portion. In the second electric wire unit 222, the (2-8)-nd electric charge 222-8 may have the smallest length.

In addition, the (3-1)-rd electrode hole 223a may be arranged in such a manner as to be positioned the farthest from the pad portion. That is, in the third electric wire 223, the (3-1)-rd electric wire 223-1 may have the greatest length. The (3-2)-rd electrode hole 223b, the (3-3)-rd electrode hole 223c, the (3-4)-rd electrode hole 223d, the (3-5)-rd electrode hole 223e, the (3-6)-rd electrode hole 223f, the (3-7)-rd electrode hole 223g, and the (3-8)-rd electrode hole 223h is arranged in such a manner as to be positioned distances away from the pad portion, and a separation distance from the pad portion may be decreased in this order. That is, the (3-8)-rd electrode hole 223h may be arranged in such a manner as to be positioned the closest to the pad portion. In the third electric wire 223, the (3-8)-rd electric wire 223-8 may have the smallest length.

In addition, the (4-1)-th electrode hole 224a may be arranged in such a manner as to be positioned the farthest from the pad portion. That is, in the fourth electric wire unit 224, the (4-1)-th electric wire 224-1 may have the greatest length. The (4-2)-th electrode hole 224b, the (4-3)-th electrode hole 224c, the (4-4)-th electrode hole 224d, the (4-5)-th electrode hole 224e, the (4-6)-th electrode hole 224f, the (4-7)-th electrode hole 224g, and the (4-8)-th electrode hole 224h are arranged in such a manner as to be positioned distances away from the pad portion, and a separation distance from the pad portion may be decreased in this order. That is, the (4-8)-th electrode hole 224h may be arranged in such a manner as to be positioned the closest to the pad portion. In the fourth electric wire unit 224, the (4-8)-th electric wire 224-8 may have the smallest length.

The (1-1)-st to (1-8)-st electric wires 221-1 to 221-8 may be arranged in such a manner as to be positioned a distance apart from each other. In the first embodiment, adjacent wires may be arranged in such a manner as to be positioned an electric-wired separation distance Wsp apart from each other. In addition, the (1-1)-st to (1-8)-st electric wires 221-1 to 221-8 may have the same width Wline. In the case, the width may be a length in the third direction (the Z-axis direction). Accordingly, the (1-1)-st to (1-8)-st electric wires 221-1 and 221-8 are electrically separated from each other. Thus, the living body signal and the living body stimulus signal may be transmitted and received, without causing an electrical short circuit, between each terminal of the circuit unit and each electrode hole brought into contact with the living body. The same may also apply to the (2-1)-nd to (2-8)-nd electric wires 222-1 to 222-8, the (3-1)-rd to (3-8)-rd electric wires 223-1 to 223-8, the (4-1)-th to (4-8)-th electric wires 224-1 to 224-8.

In addition, a plurality of electrode holes may be arranged in such a manner as to be positioned a distance apart from each other. In the first embodiment, the plurality of electrode holes included in different electric wire units may be arranged in such a manner as to be positioned a distance apart in the first direction (the X-axis direction). Therefore, the plurality of electrode holes may be arranged in such a manner as to be positioned a distance apart from each other in a length direction (the first direction) and may be sequentially arranged in a successive manner along the thickness direction (the second direction). For example, the (1-1)-st electrode hole 221a in the first electric wire unit 221 may be arranged in such a manner as to be positioned away from the (2-1)-nd electrode hole 221b, the (3-1)-rd electrode hole 221c, and the (4-1)-th electrode hole 221d. In this case, the (1-1)-st electrode hole 221a, the (2-1)-nd electrode hole 221b, the (3-1)-rd electrode hole 221c, and the (4-1)-th electrode hole 221d may be sequentially arranged in the first direction (the X-axis direction) and may overlap in the first direction (the X-axis direction). However, (1-1)-st electrode hole 221a, the (2-1)-nd electrode hole 221b, the (3-1)-rd electrode hole 221c, and the (4-1)-th electrode hole 221d may be arranged in such a manner as to be positioned a distance apart from each other in the second direction (the Y-axis direction) that is the thickness direction. In other words, the (1-1)-st electrode hole 221a, the (2-1)-nd electrode hole 221b, the (3-1)-rd electrode hole 221c), and the (4-1)-th electrode hole 221d may be stacked on top of each other in the second direction (the Y-axis direction) and thus may be arranged.

In addition, the (1-1)-st electrode hole 221a, the (2-1)-nd electrode hole 221b, (the (3-1)-rd electrode hole 221c, and the (4-1)-th electrode hole 221d may be arranged in such a manner as to be positioned the same hole separation distance Ls apart. The (1-1)-st electrode hole 221a, the (2-1)-nd electrode hole 221b, the (3-1)-rd electrode hole 221c, and the (4-1)-rd electrode hole 221d may have the same hole length Le in the first direction (the X-axis direction).

The plurality of electric holes included in the same electric wire unit may be arranged in such a manner as to be positioned a distance apart in the first direction (the X-axis direction). In this case, the separation distance that the plurality of electrode holes are positioned apart from each other may be changed in a manner that corresponds to the number of the electric wire units. For example, the (2-1)-nd electrode hole 221b, the (3-1)-rd electrode hole 221c, and the (4-1)-th electrode hole 221d may be arranged between the (1-1)-st electrode hole 221a and the (1-2)-st electrode hole 221b. In other words, respective holes in the four electric wire units are arranged between a first end portion of the (1-1)-st electrode hole 221a and a first end portion of the (1-2)-st electrode hole 221b, and thus a separation distance between the (1-1)-st electrode hole 221a and the (1-2)-st electrode hole 221b may be four times the sum of the hole length Le and the hole separation distance Ls. Regarding the separation distance, the same may apply to (1-2)-st to (1-8)-st electrode holes 221b to 221h, as well as the (1-1)-st electrode hole 221a and the (1-2)-st electrode hole 221b. In addition, the same may also apply to a separation distance between adjacent electrode holes in the (2-1)-nd to (2-8)-nd electrode holes 222a to 222h, the (3-1)-rd to (3-8)-rd electrode holes 223a and 223h, and the (4-1)-th to (4-8)-th electrode holes 224a to 224h.

In addition, the electric wire unit 220 may have a predetermined thickness d, and each electric wire unit may also have a wireless-wire thickness. In the first embodiment, the first to fourth electric wire units 221 to 224 may all have the same thickness. The first electric wire unit 221 may have a first thickness d1, the second electric wire unit 222 may have a second thickness d2, the third electric wire 223 may have a third thickness d3, and the fourth electric wire unit 224 may have a fourth thickness d4.

In the first embodiment, an entire thickness of a first end portion of the plurality of electric wires of each electric wire unit may be smaller than an entire thickness of a second end portion of the plurality of electric wires. In other words, the entire thickness of the second end portion of the plurality of electric wires may be greater than the first end portion of the plurality of electric wires. The reason for this configuration is because a first end portion of (1-1)-st electric wire 221-1 is positioned a distance away from a first end portion of the (2-1)-nd electric wire 222-1 in the length direction. Moreover, the reason for the configuration is because a first end portion of the (2-1)-nd electric wire 222-1 is positioned a distance apart from a first end portion of the (3-1)-rd electric wire 223-1 in the length direction and because a first end portion of the (3-1)-rd electric wire 223-1 is positioned a distance apart from a first end portion of the (4-1)-th electric wire 224-1 in the length direction.

However, in the first embodiment, a maximum thickness of the plurality of electric wires may be arranged adjacent to a first end portion thereof rather than a second end portion thereof. That is, in the plurality of electric wire units, electric wires may be sequentially in a successive manner in the thickness direction. Accordingly, the plurality of electric wires are partitioned into a first thickness section having a maximum thickness and a second thickness section not having the maximum thickness. In this case, a length of the first thickness section may be greater than that of the second thickness section. Therefore, a section having a maximum thickness, of the plurality of electric wires may be maximized, and thus electric resistance in the plurality of electric wire units can be maximally reduced.

As described above, the first to fourth electric wire units 221 and 224 may be stacked on top of each other in one direction (for example, the second direction). In the first to fourth electric wire units 221 and 224, electrode holes may not overlap in the second direction (the Y-axis direction). Accordingly, an electric short circuit between adjacent electrode holes can be easily blocked from ocurring.

In addition, the plurality of electrode holes in each electric wire unit may overlap in the first direction (the X-axis direction). For example, in the first electric wire unit 221, the (1-1)-st to (1-8)-st electrode holes 221a and 221h may be arranged in such a manner as to overlap in the first direction (the X-axis direction). However, as described above, the (1-1)-st to (1-8)-st electrode holes 221a and 221h may be arranged in such a manner as to be positioned a distance apart in the third direction (the Z-axis direction) and thus may be electrically separated from each other. However, in each electric wire unit, the plurality of electrode holes are arranged a predetermined separation distance apart, with the same thickness being maintained, and thus a thickness of the electric wire unit may be maximized at a maximally close position. With this configuration, a width of each electric wire unit may be reduced, and a length of a section having a maximum width may be reduced. In other words, although the electric wire units have the same length, the length of the section having the maximum width may be reduced, and thus resistance may be reduced. Thus, the electric resistance is reduced in the electric resistance is reduced, and thus the electrical efficiency of the implantable device can be improved. In addition, an amount of heat can be reduced, and thus the reliability of the implantable device can be improved.

In the first embodiment, a shape of the substrate portion may vary with a width, thickness, and length of the electric wire unit 220. A first end portion of the electric wire unit may have a smaller width than a second end portion thereof. The second end portion of the electric wire unit may be arranged to be more adjacent to the pad portion than the first end portion thereof. That is, a first end portion of the substrate portion may have a minimum width W1, and a second end portion thereof may have a maximum width W2. In this case, the minimum width W1 may be smaller than the maximum width W2, and a portion with the maximum width W2 may be positioned under a portion with the minimum width W1.

In addition, the more approached the pad portion, the more a width of the electric wire unit 220 may be increased. A maximum width may be maintained from a certain section up to the pad portion. Specifically, a maximum width of the first electric wire unit 221 may be maintained from a first end portion of the (1-8)-st electrode hole 221h to a second end portion of the (4-8)-th electrode hole 224h.

Likewise, the substrate portion may have a first section S1 having a maximum width and a second section S2 not having the maximum width. In the second section S2, a width may be gradually reduced in the second direction (the Y-axis direction). In the first section S1, the maximum width may be maintained.

That is, a length of the first section S1 of the substrate portion may be smaller than a length Lm of the second section S2 thereof. In other words, a section having the maximum width is arranged adjacent to the pad portion, and thus the width of the electric wire unit is reduced. Accordingly, the electrical resistance can be reduced. Therefore, advantages of improving the electrical efficiency of the implantable device and reducing the amount of heat dissipated through the electric wire can be provided.

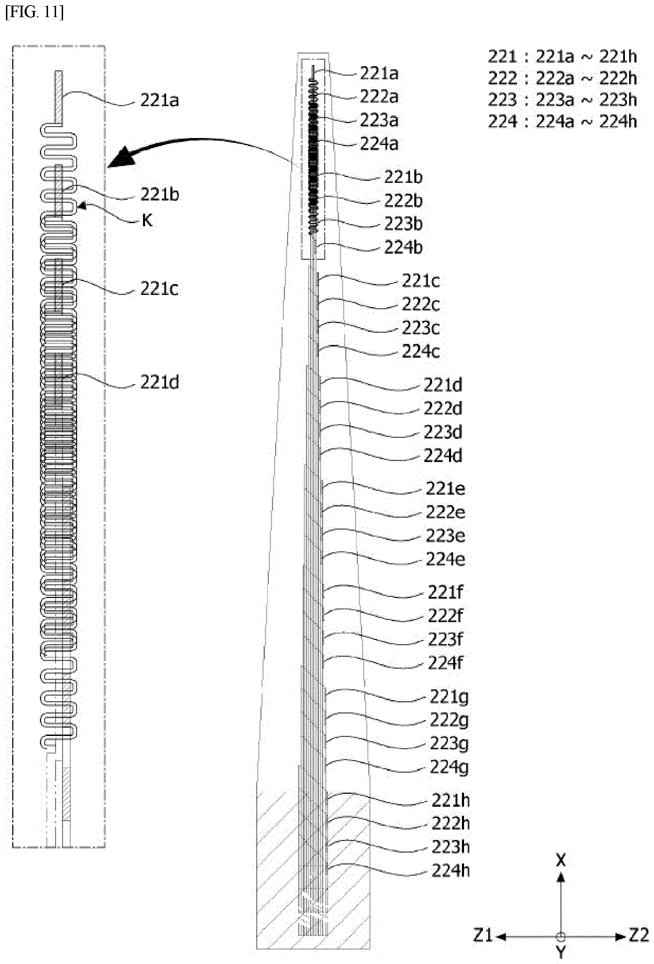

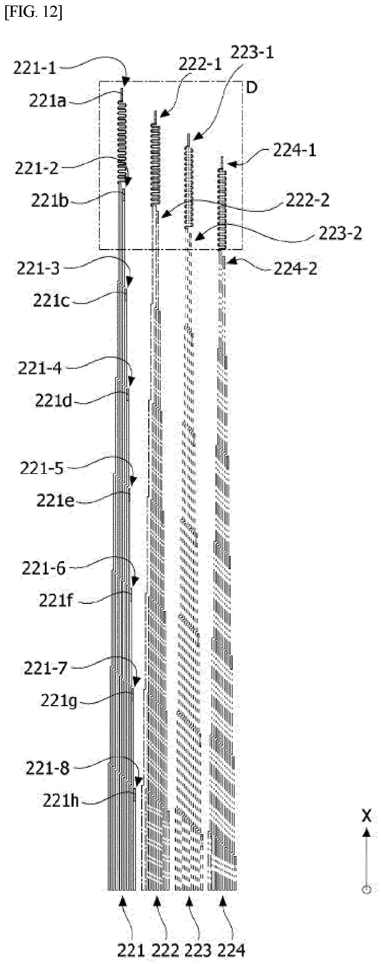

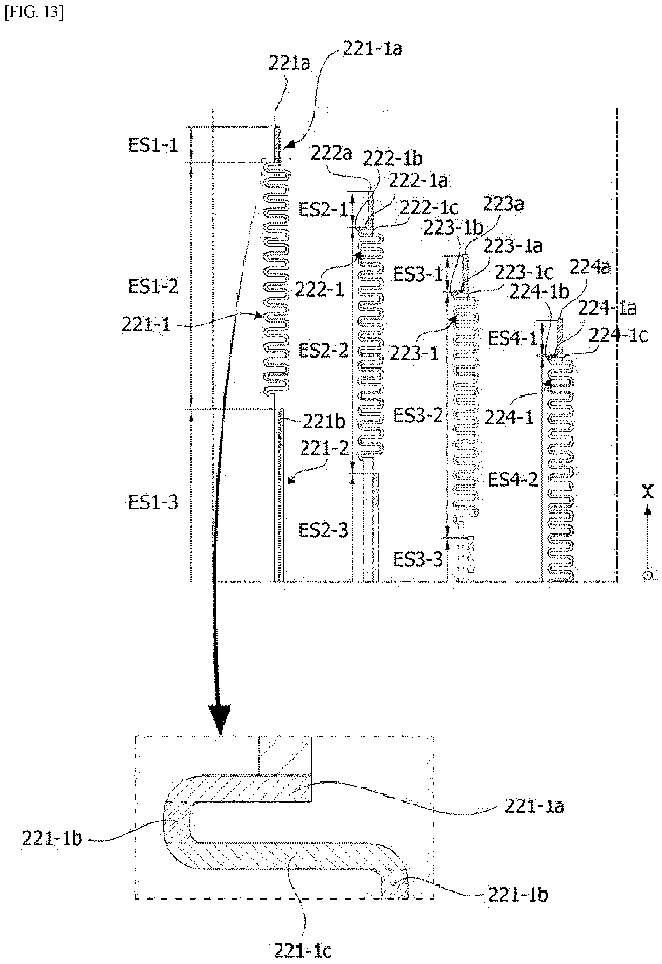

FIG. 11 is a view illustrating an electric wire within an electric wire unit according to a second embodiment of the present invention. FIG. 12 is a plane view illustrating each electric wire within the electric wire array according to the second embodiment. FIG. 13 is an enlarged view illustrating portion D in FIG. 12.

With reference to FIGS. 11 and 13, the electric wire within the electric wire array according to the second embodiment is the same as the electric wire within the electric wire array according to the first embodiment, except for the following description. The same description is not repeated.

Specifically, in the second embodiment, the electric wire unit 220 may include the first electric wire unit 221, the second electric wire unit 222, the third electric unit 223, and the fourth electric wire unit 224. The number of the electric wire units 220 may correspond to the number of channels of the circuit unit. For example, the circuit unit may have a plurality of terminals performing different functions, as one channel. A plurality of channels, each serving as the plurality of terminals, may be provided. In the first embodiment, in a case where the number of the channels of the circuit unit is 4, the electric wire unit 220, as described above, may include the first to fourth electric wire units 221 to 224. A description is provided below based on this case. Each electric wire unit may include a plurality of electric wires. In the present specification, a description is provided on the assumption that there are 8 electric wires in each electric wire unit.

Specifically, the first to fourth electric wire units 221 and 224 each may have 8 electric wires. Accordingly, the first electric wire unit 221 may include (1-1)-st to (1-8)-st electric wires 221-1 to 221-8. Likewise, the second electric wire unit 222 may include (2-1)-nd to (2-8)-nd electric wires 222-1 to 222-8. The third electric wire 223 may include (3-1)-rd to (3-8)-rd electric wires 223-1 and 223-8. The fourth electric wire unit 224 may include (4-1)-th to (4-8)-th electric wires 224-1 and 224-8.

The above-described electrode hole may be arranged in a first end portion of each electric wire. Each electric wire may be arranged in such a manner as to extend from each electrode hole to the pad portion. In addition, each electrode hole, as described above, is brought into contact with the living body. Therefore, a contact portion protruding in the second direction (the Y-axis direction) or the third direction (the Z-axis direction) may be additionally arranged in each electrode hole. The same may apply to the above-described first embodiment.

In the second embodiment, the (1-1)-st electric wire 221-1 may include the (1-1)-st electrode hole 221a. The (1-2)-st electric wire 221-2 may include the (1-2)-st electrode hole 221b. The (1-3)-st electric wire 221-3 may include the (1-3)-st electrode hole 221c. The (1-4)-st electric wire 221-4 may include the (1-4)-st electrode hole 221d. The (1-5)-st electric wire 221-5 may include the (1-5)-st electrode hole 221e. The (1-6)-st electric wire 221-6 may include the (1-6)-st electrode hole 221f The (1-7)-st electric wire 221-7 may include the (1-7)-st electrode hole 221g. The (1-8)-st electric wire 221-8 may include the (1-8)-st electrode hole 221h.

Likewise, the (2-1)-nd electric wire 222-1 may include the (2-1)-nd electrode hole 222a, the (2-2)-nd electric wire 222-2 may include the (2-2)-nd electrode hole 222b. The (2-3)-nd electric wire 222-3 may include the (2-3)-nd electrode hole 222c. The (2-4)-nd electric wire 222-4 may include the (2-4)-nd electrode hole 222d. The (2-5)-nd electric wire 222-5 may include the (2-5)-nd electrode hole 222e. The (2-6)-nd electric wire 222-6 may include the (2-6) electrode hole 222f The (2-7)-nd electric wire 222-7 may include the (2-7)-nd electrode hole 222g. The (2-8)-nd electric charge 222-8 may include the (2-8)-nd electrode hole 222h.

The (3-1)-rd electric wire 223-1 may include the (3-1)-rd electrode hole 223a. The (3-2)-rd electric wire 223-2 may include the (3-2)-rd electrode hole 223b. The (3-3)-rd electric wire 223-3 may include the (3-3)-rd electrode hole 223c. The (3-4)-rd electric wire 223-4 may include the (3-4)-rd electrode hole 223d. The (3-5)-rd electric wire 223-5 may include the (3-5)-rd electrode hole 223e. The (3-6)-rd electric wire 223-6 may include the (3-6)-rd electrode hole 223f The (3-7)-rd electric wire 223-7 may include the (3-7)-rd electrode hole 223g. The (3-8)-rd electric wire 223-8 may include the (3-8)-rd electrode hole 223h.

The (4-1)-th electric wire 224-1 may include the (4-1)-th electrode hole 224a. The (4-2)-th electric wire 224-2 may include the (4-2)-th electrode hole 224b. The (4-3)-th electric wire 224-3 may include the (4-3)-th electrode hole 224c. The (4-4)-th electric wire 224-4 may include the (4-4)-th electrode hole 224d. The (4-5)-th electric wire 224-5 may include the (4-5)-th electrode hole 224e. The (4-6)-th electric wire 224-6 may include the (4-6)-th electrode hole 224f The (4-7)-th electric wire 224-7 may include the (4-7)-th electrode hole 224g. The (4-8)-th electric wire 224-8 may include the (4-8)-th electrode hole 224h.

The electrode hole arranged in each electric wire may be positioned in a first end portion thereof, which is an outermost portion in the first direction (the X-axis direction). That is, the (1-1)-st electrode hole 221a may be arranged in such a manner as to be positioned the farthest away from the pad portion. The (1-1)-st electric wire 221-1 in the first electric wire unit 221 may have the greatest length. The length here means a length in the first direction (the X-axis direction). The (1-2)-st electrode hole 221b, the (1-3)-st electrode hole 221c, the (1-4)-st electrode hole 221d, the (1-5)-st electrode hole 221e, the (1-6)-st electrode hole 221f, the (1-7)-st electrode hole 221g, and the (1-8)-st electrode hole 221h are arranged in such a manner as to be positioned distances away from the pad portion, and a separation distance from the pad portion may be decreased in this order. That is, the (1-8)-st electrode hole 221h may be arranged in such a manner as to be positioned the closest to the pad portion. In the first electric wire unit 221, the (1-8)-st electric wire 221-8 may have the smallest length.

Likewise, the (2-1)-nd electrode hole 222a may be arranged in such a manner as to be positioned the farthest away from the pad portion. That is, in the second electric wire 222, the (2-1)-rd electric wire 222-1 may have the greatest length. The (2-2)-nd electrode hole 222b, the (2-3)-nd electrode hole 222c, the (2-4)-nd electrode hole 222d, the (2-5)-nd electrode hole 222e, the (2-6) electrode hole 222f, the (2-7)-nd electrode hole 222g, and the (2-8)-nd electrode hole 222h are arranged in such a manner as to be positioned distances away from the pad portion, and a separation distance from the pad portion may be decreased in this order. That is, the (2-8)-nd electrode hole 222h may be arranged in such a manner as to be positioned the closest to the pad portion. In the second electric wire unit 222, the (2-8)-nd electric charge 222-8 may have the smallest length.

In addition, the (3-1)-rd electrode hole 223a may be arranged in such a manner as to be positioned the farthest from the pad portion. That is, in the third electric wire 223, the (3-1)-rd electric wire 223-1 may have the greatest length. The (3-2)-rd electrode hole 223b, the (3-3)-rd electrode hole 223c, the (3-4)-rd electrode hole 223d, the (3-5)-rd electrode hole 223e, the (3-6)-rd electrode hole 223f, the (3-7)-rd electrode hole 223g, and the (3-8)-rd electrode hole 223h is arranged in such a manner as to be positioned distances away from the pad portion, and a separation distance from the pad portion may be decreased in this order. That is, the (3-8)-rd electrode hole 223h may be arranged in such a manner as to be positioned the closest to the pad portion. In the third electric wire 223, the (3-8)-rd electric wire 223-8 may have the smallest length.

In addition, the (4-1)-th electrode hole 224a may be arranged in such a manner as to be positioned the farthest from the pad portion. That is, in the fourth electric wire unit 224, the (4-1)-th electric wire 224-1 may have the greatest length. The (4-2)-th electrode hole 224b, the (4-3)-th electrode hole 224c, the (4-4)-th electrode hole 224d, the (4-5)-th electrode hole 224e, the (4-6)-th electrode hole 224f, the (4-7)-th electrode hole 224g, and the (4-8)-th electrode hole 224h are arranged in such a manner as to be positioned distances away from the pad portion, and a separation distance from the pad portion may be decreased in this order. That is, the (4-8)-th electrode hole 224h may be arranged in such a manner as to be positioned the closest to the pad portion. In the fourth electric wire unit 224, the (4-8)-th electric wire 224-8 may have the smallest length.

The (1-1)-st to (1-8)-st electric wires 221-1 to 221-8 may be arranged in such a manner as to be positioned a distance apart from each other. In the second embodiment, adjacent electric wires, as described above, may be arranged in such a manner as to be positioned a predetermined electric-wire separation distance apart from each other. In addition, the (1-1)-st to (1-8)-st electric wires 221-1 to 221-8 each may have the same width. In the case, the width may be a length in the third direction (the Z-axis direction). Accordingly, the (1-1)-st to (1-8)-st electric wires 221-1 and 221-8 are electrically separated from each other. Thus, the living body signal and the living body stimulus signal may be transmitted and received, without causing an electrical short circuit, between each terminal of the circuit unit and each electrode hole brought into contact with the living body. The same may also apply to the (2-1)-nd to (2-8)-nd electric wires 222-1 to 222-8, the (3-1)-rd to (3-8)-rd electric wires 223-1 to 223-8, the (4-1)-th to (4-8)-th electric wires 224-1 to 224-8.

In addition, the plurality of electrode holes may be arranged in such a manner as to be positioned a distance apart from each other. In the second embodiment, a plurality of electrode holes included in the same electric wire units or in different electric wire units may be arranged in such a manner as to be positioned a distance apart in the first direction (the X-axis direction). Additionally, a plurality of electrode holes in different electric wire units may be arranged in such a manner as to be positioned a distance apart in the second direction (the Y-axis direction).

For example, the (1-1)-st electrode hole 221a, the (2-1)-nd electrode hole (222a), the (3-1)-rd electrode hole 223a, and the (4-1)-th electrode hole 224a may be arranged in such a manner as to be positioned a distance apart in the first direction (the X-axis direction). Moreover, the (1-1)-st electrode hole 221a, the (2-1)-nd electrode hole 222a, the (3-1)-rd electrode hole 223a, and the (4-1)-th electrode hole 224a may be arranged in such a manner as to be positioned a distance apart in the second direction (the Y-axis direction). However, the (1-1)-st electrode hole 221a, the (2-1)-nd electrode hole (222a), the (3-1)-rd electrode hole 223a, and the (4-1)-th electrode hole 224a may be arranged in such a manner as to overlap in the first direction (the X-axis direction).

In addition, the (1-1)-st to (1-8)-st electrode holes 221a to 221h may be arranged in such a manner as to be positioned a distance apart in the first direction (the X-axis direction) or the 3-third direction (the Z-axis direction). However, the (1-1)-st to (1-8)-st electrode holes 221a to 212h may be arranged at the same height in the second direction (the Y-axis direction).

In addition, a plurality of electrode holes may be arranged in such a manner as to be positioned a distance apart from each other in the length direction (the first direction), and may be sequentially arranged in a successive manner along the thickness direction (the second direction).

That is, the (1-1)-st electrode hole 221a, the (2-1)-nd electrode hole 221b, the (3-1)-rd electrode hole 221c, and the (4-1)-th electrode hole 221d may be sequentially arranged along the first direction (the X-axis direction) and may overlap in the first direction (the X-axis direction). However, as described above, the (1-1)-st electrode hole 221a, the (2-1)-nd electrode hole 221b, the (3-1)-rd electrode hole 221c, and the (4-1)-th electrode hole 221d may be arranged in such a manner as to be positioned a distance apart from each other in the second direction that is the thickness direction. In other words, the (1-1)-st electrode hole 221a, the (2-1)-nd electrode hole 221b, the (3-1)-rd electrode hole 221c), and the (4-1)-th electrode hole 221d may be stacked on top of each other in the second direction (the Y-axis direction) and thus may be arranged.

In addition, the (1-1)-st electrode hole 221a, the (2-1)-nd electrode hole 221b, the (3-1)-rd electrode hole 221c, and the (4-1)-th electrode hole 221d may be all positioned the same separation distance away from the electrode hole adjacent in the first direction (the X-axis direction). However, no limitation to this separation distance is imposed.

The separation distance that the plurality of electrode holes included in the same electric wire unit are positioned apart from each other may be changed in a manner that corresponds to the number of the electric wire units. For example, the (2-1)-nd electrode hole 221b, the (3-1)-rd electrode hole 221c, and the (4-1)-th electrode hole 221d may be arranged between the (1-1)-st electrode hole 221a and the (1-2)-st electrode hole 221b. In other words, respective holes in the four electric wire units are arranged between a first end portion of the (1-1)-st electrode hole 221a and a first end portion of the (1-2)-st electrode hole 221b, and thus the separation distance between the (1-1)-st electrode hole 221a and the (1-2)-st electrode hole 221b may be four times the sum of the hole length and the hole separation distance. Regarding the separation distance, the same may apply to (1-2)-st to (1-8)-st electrode holes 221b to 221h, as well as the (1-1)-st electrode hole 221a and the (1-2)-st electrode hole 221b. However, no limitation to this separation distance is imposed.

In addition, the same may also apply to a separation distance between adjacent electrode holes in the (2-1)-nd to (2-8)-nd electrode holes 222a to 222h, the (3-1)-rd to (3-8)-rd electrode holes 223a and 223h, and the (4-1)-th to (4-8)-th electrode holes 224a to 224h.

Furthermore, the electric wire unit 220 may have a predetermined thickness, and each electric wire unit may also have a wireless-wire thickness. In the second embodiment, the first to fourth electric wire units 221 to 224 may all have the same thickness.

In addition, each first electric wire in each electric wire unit may include a protrusion portion K protruding in the third direction (the Z-axis direction) or the second direction (the Y-axis direction) between the first electrode hole and the second electrode hole. That is, each first electric wire in each electric wire unit may have a concave-convex structure between the first electrode hole and the second electrode hole. Each first electric wire will be described below as having the concave-convex structure in the third direction (the Z-axis direction).

First, first electric wires 221-1, 222-1, 223-1, and 224-1 in each electric wire unit may include first electric wire sections ES1-1, ES2-1, ES3-1, and ES4-1, second electric wire sections ES1-2, ES2-2, ES3-2, and ES4-2, and third electric wire sections ES1-3, ES2-3, ES3-3, and ES4-3, respectively. The first electrode holes 221a, 222a, 223a, and 224a are positioned in the first electric wire sections ES1-1, ES2-1, ES3-1, and ES4-1, respectively, in the first direction (the X-axis direction). The second electric wire section ES1-2 is positioned between the first electrode holes 221a and the second electrode holes 221b. The second electric wire section ES2-2 is positioned between the first electrode holes 222a and the second electrode holes 222b. The second electric wire section ES3-2 is positioned between the first electrode holes 223a and the second electrode holes 223b. The second electric wire section ES4-2 is positioned between the first electrode holes 224a and the second electrode holes 224b. The third electric wire section ES1-3 is other than the first electric wire sections ES1-1 and the second electric wire sections ES1-2. The third electric wire section ES2-3 is other than the first electric wire sections ES2-1 and the second electric wire sections ES2-2. The third electric wire section ES3-3 is other than the first electric wire sections ES3-1 and the second electric wire sections ES3-2. The third electric wire section ES4-3 is other than the first electric wire sections ES4-1 and the second electric wire sections ES4-2.

That is, the first electric wire sections ES1-1, ES2-1, ES3-1, and ES4-1 may overlap the first electrode holes 221a, 222a, 223a, and 224a, respectively, in the second direction (the Y-axis direction or the third direction (the Z-axis direction). The third electric wire section ES1-3 may overlap an electrode hole other than the first electrode hole 221a in the second direction or the third direction. The third electric wire section ES2-3 may overlap an electrode hole other than the first electrode hole 222a in the second direction or the third direction. The third electric wire section ES3-3 may overlap an electrode hole other than the first electrode hole 223a in the second direction or the third direction. The third electric wire section ES4-3 may overlap an electrode hole other than the first electrode hole 224a in the second direction or the third direction.

For example, the second electric wire section may include a (2-1)-nd electric wire section ES1-2, a (2-2)-nd electric wire section ES2-2, a (2-3)-nd electric wire section ES3-2, and a (2-4)-nd electric wire section ES4-2. The (2-1)-nd electric wire section ES1-2 may be positioned within the (1-1)-st electric wire 221-1. The (2-2)-nd electric wire section ES2-2 may be positioned within the (2-1)-nd electric wire 222-1. The (2-3)-nd electric wire section ES3-2 may be positioned within the (2-3)-nd electric wire 223-1. The (2-4)-nd electric wire section ES4-2 may be positioned within the (2-4)-nd electric wire 224-1.

In the second embodiment, each first electric wire may have the protrusion portion K in the second electric wire sections ES1-2, ES2-2, ES3-2, and ES4-2. More specifically, the first electric wires 221-1, 222-1, 223-1, and 224-1 may include first extension portions 221-1a, 222-1a, 223-1a, and 224-1a, second extension portions 221-1b, 222-1b, 223-1b, and 224-1b, and third extension portions 221-1c, 222-1c, 223-1c, and 224-1c in the second electric wire sections ES1-2, ES2-2, ES3-2, and ES4-2, respectively. The first extension portions 221-1a, 222-1a, 223-1a, and 224-1a extend in a (3-1)-rd direction (a Z1 direction). The second extension portions 221-1b, 222-1b, 223-1b, and 224-1b extend the first direction. The third extension portions 221-1c, 222-1c, 223-1c, and 224-1c extend in a (3-2)-rd direction (a Z2 direction).

In the second embodiment, the first extension portion 221-1a, the second extension portion 221-1b and the third extension portion 221-1c, which extend, may be connected to each other sequentially or randomly. The first extension portion 222-1a, the second extension portion 222-1b and the third extension portion 222-1c, which extend, may be connected to each other sequentially or randomly. The first extension portion 223-1a, the second extension portion 223-1b and the third extension portion 223-1c, which extend, may be connected to each other sequentially or randomly. The first extension portion 224-1a, the second extension portion 224-1b and the third extension portion 224-1c, which extend, may be connected to each other sequentially or randomly. In this case, the second extension portions 221-1b, 222-1b, 223-1b, and 224-1b and the third extension portions 221-1c, 222-1c, 223-1c, and 224-1c all extend in the third direction. However, in the present specification, a description is provided on the assumption of a structure where an electric wire is connected in a direction from a first end portion to a second end portion.

In other words, with the first extension portions 221-1a, 222-1a, 223-1a, and 224-1a,the second extension portions 221-1b, 222-1b, 223-1b, and 224-1b, and the third extension portions 221-1c, 222-1c, 223-1c, and 224-1c, the first electric wire may include the protrusion portion protruding from the second electric wire sections ES1-2, ES2-2, ES3-2, and ES4-2 toward the (3-1)-rd direction (the Z1 direction) or the (3-2)-nd direction (the Z2 direction). The protrusion portions extending in the (3-1)-rd direction (the Z1) direction or the (3-2)-rd direction may also be positioned in given order along the first direction (the X-axis direction).

Accordingly, with the arrangement of the first extension portions 221-1a, 222-1a, 223-1a, and 224-1a,the second extension portions 221-1b, 222-1b, 223-1b, and 224-1b, and the third extension portions 221-1c, 222-1c, 223-1c, and 224-1c, the first electric wires 221-1, 222-1, 223-1, and 224-1 each may include the protrusion portion protruding in the third direction (the Z-axis direction).

In this case, the protrusion portion K, as described above, may be arranged in the respective second electric wire sections ES1-2, ES2-2, ES3-2, and ES4-2 of the first electric wires 221-1, 222-1, 223-1, and 224-1 in each electric wire unit.

Therefore, the flexibilities of the respective first electrode holes 221a, 222a, 223a, and 224a that are arranged in outermost portions or apexes of the electric wires may be maximized by the second electric wire sections ES1-2, ES2-2, ES3-2, and ES4-2, respectively, that have a zigzag shape or the like. Thus, when the implantable device is implanted into the living body, the electrode hole in the electric wire may be easily arranged deep into the living body. Moreover, with the second electric wire sections (ES1-2, ES2-2, ES3-2, and ES4-2), the electric wire array according to the second embodiment blocks a crack or the like from occurring in each electric wire unit. Thus, the reliability of the electric wire unit can be improved. Accordingly, the flexibility of the electrode hole arranged deep into the living body can be increased. Furthermore, the length of the electric wire having the other electrode holes can be minimized, and thus the electric resistance can be minimized.

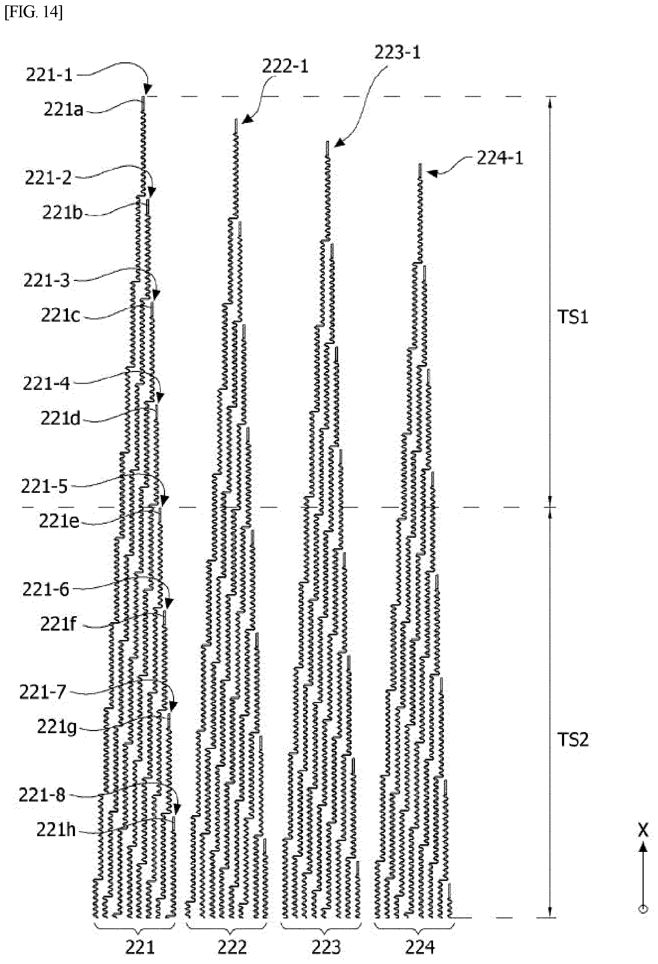

FIG. 14 is a view illustrating an electric wire within an electric wire array according to a third embodiment of the present invention.

With reference to FIG. 14, each electric wire in each electric wire unit within the electric wire array according to the third embodiment may include the above-described protrusion portion in a section not having the electrode hole.

For example, the (1-1)-st electric wire 221-1 may include the protrusion portion in a section other than the (1-1)-st electrode hole 221a. That is, as described with reference to FIGS. 11 to 13, the (1-1)-st electric wire 221-1 may include the protrusion portion in the second electric wire section and the 3-third electric wire section.

In addition, the (1-2)-st electric wire 221-2 may include a protrusion portion in a section other than the (1-2)-st electrode hole 221b. The (1-3)-st electric wire 221-3 may include a protrusion portion in a section other than the (1-3)-st electrode hole 221c. The (1-4)-st electric wire 221-4 may include a protrusion portion in a section other than the (1-4)-st electrode hole 221d. The (1-5)-st electric wire 221-5 may include a protrusion portion in a section other than the (1-5)-st electrode hole 221e. The (1-6)-st electric wire 221-6 may include a protrusion portion in a section other than the (1-6)-st electrode hole 221f The (1-7)-st electric wire 221-7 may include a protrusion portion in a section other than the (1-7)-st electrode hole 221g. The (1-8)-st electric wire 221-8 may include a protrusion portion in a section other than the (1-8)-st electrode hole 221h.

The same may apply to a plurality of second electric wires 222-1 to the (2-8)-nd electric wire of the second electric wire unit 222, the (3-1)-rd to (3-8)-rd electric wires 223-1 and 223-8 of the third electric wire 223, and the (3-1)-rd to (3-8)-rd electric wires 224-1 to 224-8 of the (4-8)-th electric wire 224-8.

With this configuration, in the electric wire array according to the third embodiment, the flexibility is provided to all the electric wires, as well as the respective first to eighth electrodes arranged in outermost portions or apexes of the electric holes. Thus, the electrode holes may be arranged deep into the living body. Moreover, the electric wire array according to the third embodiment may block a crack or the like from occurring in each electric wire unit, and thus the reliability of the electric wire unit can be improved.

In addition, as a modification example, each of the electric wire units 221 to 224 may include the protrusion portion only within the section resulting from partitioning in the first direction (the X-axis direction). Particularly, the protrusion portion may be arranged in a section including the apex, of each of the electric wire units 221 to 224.

Specifically, the first electric wire unit 221 may be partitioned, by a bisector C1 having a maximum length, in the first direction (the X-axis direction), into a first division section TS1 and a second division section TS2. In this case, a length in the first direction (the X-axis direction), of the first division section TS1 may be greater than a length in the first direction (the X-axis direction), of the second division section TS.

As a modification example, the first electric wire unit 221 may include the protrusion portion in the first division section TS1. The protrusion portion may not be arranged in the second division section TS2. Thus, the first division section TS1 brought into contact with a living-body portion deep in the living body has the protrusion portion, and thus can have the flexibility. Furthermore, the second division section TS2 can provide an electric property (resistance reduction) improved by reducing a width in the third direction of the electric wire. The embodiments are described above in a focused manner only for illustrative purpose and does not impose any limitation on the present invention. It would be understood by a person of ordinary skill in the art to which the present invention pertains that various embodiments and applications, which are not described above, may be possible without departing the scope of the essential features of the embodiments. For example, each of the constituent elements realized in the embodiments may be modified and then implemented. Differences associated with these modifications and applications should be interpreted as falling within the scope of the present invention that is defined by the following claims.

## Claims

1. An electric wire array comprising:
a substrate portion; and
a plurality of electric line units being arranged on top of the substrate portion and being stacked on top of each other in a thickness direction,
wherein the plurality of electric wire units each comprise:
a plurality of electric wires being arranged on top of the substrate portion in such a manner to extend in a length direction; and
a plurality of electrode holes being positioned in respective first end portions, respectively, of the plurality of electric wires, and being exposed,
wherein the plurality of electrode holes are arranged in such a manner as to be positioned a distance apart from each other along the length direction and being sequentially arranged in a successive manner along the thickness direction, and
wherein the thickness direction is a direction from the substrate portion to the electric wire unit, and the length direction is a direction vertical to the thickness direction.

2. The electric wire array of claim 1, wherein an entire thickness of a first end portion of the plurality of electric wires is smaller than an entire thickness of a second end portion of the plurality of electric wires.

3. The electric wire array of claim 2, wherein a section with a maximum width, of the plurality of electric wires is positioned more adjacent to the second end portion than to the first end portion.

4. The electric wire array of claim 1,
wherein the plurality of electric wire units each comprise:
a first electric wire unit; and
a second electric wire unit arranged on top of the first electric wire unit,
wherein the first electric wire unit comprises a plurality of first electric wires having a plurality of first electrode holes, respectively, and
wherein the second electric wire unit comprises a plurality of second electric wires having a plurality of second electrode holes, respectively.

5. The electric wire array of claim 4, wherein the plurality of first electrode holes overlap in the length direction, and the plurality of second electrode holes overlap in the length direction.

6. The electric wire array of claim 4, wherein the plurality of first electric wires are arranged in such a manner as to be positioned a distance apart from each other, and the plurality of second electric wires are arranged in such a manner as to be positioned a distance apart from each other.

7. The electric wire array of claim 1, wherein an entire thickness of a first end portion of the plurality of electric wires is smaller than an entire thickness of a second end portion of the plurality of electric wires.

8. The electric wire array of claim 7,
wherein the plurality of electric wires comprise:
a first thickness section having a maximum thickness; and
a second thickness section other than the first thickness section,
wherein a length of the first thickness section is greater than a length of the second thickness section.

9. The electric wire array of claim 1,
wherein the plurality of electric wires include
a (1-1)-st electric wire; and
a (1-2)-st electric wire having small length in the length direction than the (1-1)-st electric wire,
wherein the (1-1)-st electric wire includes a (1-1)-st electrode hole, and the (1-2)-st electric wire includes a (1-2) electrode hole,
wherein the (1-1)-st electric wire includes a protrusion portion between the (1-1)-st electrode hole and the (1-2)-st electrode hole, the protrusion portion protruding in the thickness direction or a width direction, and
wherein the width direction is a direction vertical to the length direction and the thickness direction.

10. An implantable device comprising:
a body portion; and
an electric wire array electrically connected to the body portion,
wherein the electric wire array comprises;
a substrate portion; and
a plurality of electric line units being arranged on top of the substrate portion and being stacked on top of each other in a thickness direction,
wherein the plurality of electric wire units each comprise:
a plurality of electric wires being arranged on top of the substrate portion in such a manner to extend in a length direction; and
a plurality of electrode holes being positioned in respective first end portions, respectively, of the plurality of electric wires and being exposed,
wherein the plurality of electrode holes are arranged in such a manner as to be positioned a distance apart from each other along the length direction and being sequentially arranged in a successive manner along the thickness direction,
wherein the body portion comprises:
pad portions electrically connected to the plurality of electric wire units, respectively; and
a circuit unit including terminals electrically connected to the pad portions, respectively, and
wherein the thickness direction is a direction from the substrate portion to the electric wire unit, and the length direction is a direction vertical to the thickness direction.
